# EUROPEAN PATENT APPLICATION

(11) **EP 2 450 012 A1**
(43) Date of publication of application: **09.05.2012**
(21) Application number: 10447020.8
(22) Date of filing: 13.10.2010
(51) Int. Cl.: A61F 13/15

(54) **Improved absorbent structure**

(71) Applicant: Romanova bvba starter, 9255 Buggenhout (BE)
(72) Inventor: van de Maele, Marleen, 9255 Buggenhout (BE)

(57) **Abstract**

The present invention relates to an absorbent structure for use in an absorbent article, preferably a disposable article, such as a feminine hygiene garment, baby diaper, baby pants and adult incontinence garment; to an absorbent article comprising such absorbent structure and to a method of manufacturing the absorbent structure. The absorbent structures according to the invention comprise a distribution layer having a minimum absorbent capacity, an immobilisation layer which joins to the distribution layer to define compartments there between; and an absorbent material held in at least one of the compartments. The present invention foresees in the need for improved thin, flexible, lightweight absorbent structures for use in absorbent articles which overcomes the liquid management problems of the prior art during absorption, distribution and retention of bodily exudates with an optimal fit and wearing comfort.

## Description

### FIELD OF THE INVENTION

The present invention relates to an absorbent structure for use in an absorbent article, preferably a disposable article, such as a feminine hygiene garment, baby diaper, baby pants and adult incontinence garment; to an absorbent article comprising such absorbent structure and to a method of manufacturing the absorbent structure.

### BACKGROUND OF THE INVENTION

Disposable absorbent articles have an absorbent structure for absorbing bodily exudates, a soft liquid-permeable top sheet on the wearer side and a liquid-impermeable back sheet on the garment side. The absorbent structure is normally made from a mixture of fluff pulp or other fibrous substance and an absorbent polymer material.

There has been increasing demand in recent years for flexible, thinner, more lightweight absorbent articles to resolve various problems of manufacturing, marketing, design, fit, wearing comfort, distribution, garbage disposal, material and energy consumption, transportation, storage costs and the like.

The most common method currently used to meet these demands in disposable absorbent articles is to reduce the amount of cellulose fibre or other support within and surrounding the absorbent core and/or to use larger amounts of absorbent polymer materials.

Such absorbent articles having a smaller proportion of hydrophilic cellulose fibres and a higher proportion of super absorbent polymers may be better at storing liquid, however they are not necessarily good at absorbing and distributing the liquid when the absorbent article is actually being used.

The ability and capacity of an absorbent polymer material to absorb, distribute and retain liquid is dependent upon the form, position and/or manner in which the absorbent polymer material is incorporated into the absorbent structure. Whenever a particle of the absorbent polymer material in an absorbent structure is wetted, it swells and forms a gel. This gel formation can block liquid transmission into the interior of the absorbent core, a phenomenon called "gel blocking". Gel blocking in and adjacent a zone in the absorbent core of initial liquid contact prevents liquid from rapidly dispersing or wicking past the "blocking" particles of absorbent polymer material into the rest of the absorbent core and further liquid uptake by the absorbent core must then take place via an alternative diffusion process that is much slower than the rate at which liquid is applied to the absorbent structure.

The apparent larger proportion of absorbent polymer materials required in substantially cellulose free absorbent articles may thus greatly inhibit the absorption, distribution and retention of liquids in case the gel blocking is not adequately prevented, minimized or compensated. Inadequate management of the absorbent polymer materials in substantially cellulose free absorbent articles results in leakages from the absorbent article well before the absorbent core is fully saturated, since the decreased absorption speed and fluid distribution will prevent the usage of available absorbent materials within the absorbent article.

It will be clear that the absolute and relative proportions of the hydrophilic cellulose fibre and absorbent polymer materials will thus need to be restricted in order to maintain the absorbent properties of the absorbent core. Hence, there are certain limits on reducing the amount of hydrophilic cellulose fibre and reducing the thickness of the absorbent core.

What is needed is an absorbent structure that quickly takes up and continuously distributes quantities of liquids throughout the absorbent core while managing gel blocking during and after liquid contact. Moreover, since such hygienic absorbent articles are generally a disposable product, it needs in some instances to be worn over many hours and worn both in a dry state as well as in a partially and fully bodily exudates loaded state.

Hence, to provide low rewet, good fit, wearing comfort and avoid prolonged skin contact in between the wearer's skin and the absorbent polymer material it is very important to keep the absorbent materials comprised by an absorbent article in its intended position, both when the article is dry as well as the absorbent article is partially or fully loaded with bodily exudates.

Multiple attempts have been made to provide the above flexible, thinner, light-weight absorbent articles comprising a substantially cellulose free absorbent structure combined with relative high proportions of absorbent polymer material having good fluid handling performance, capabilities and comfort which include:
EP0844859 discloses an absorbent article with an absorbent structure for the absorption of liquids comprising a pair of outer flat non-woven fabric layers of thermoplastic fibres which are heat and calendar sealed together along a plurality of intersecting bonding lines to form permanent individually closed non-extendible pockets containing absorbent particulate polymer material. By the absence of hydrophilic cellulose fibres, the absorbent article is solely relying on the communication of the absorbent polymer material contained within the pockets which are spatially separated from and confined within the permanent thermoplastic grid structure to accommodate liquid absorption, distribution and retention. Hence, when the absorbent article is fully or partially loaded with bodily exudates said thermo-pressure fused bonds functionally and structurally separate the absorbent polymer materials containing pockets from one another, which furthermore increase the overall stiffness and rigidity of the absorbent structure, and may severely hinder efficient and effective usage of the available absorbent polymer material due to limited fluid dispersion and transport from the less absorbent areas to the more absorbent areas of the absorbent structure thereby procuring gel blocking, higher rewet values and frequent leakage. Therefore, it is believed that this absorbent article does not provide a very satisfactory substantially cellulose free concept in either fully or partially bodily exudates loaded state.
US5643238 discloses an absorbent article with an absorbent structure comprising storage cells and acquisition cells in a grid configuration. Within the storage cells of the absorbent structure a quantity of absorbent polymer material is deposited while the spaced apart acquisition cells are devoid of any absorbent material. According to the teaching of the patent the acquisition cells function as channels that allow liquid transport within the absorbent structure to help prevent gel blocking. However, due to the absence of absorbent materials within these acquisition cells and the relatively limited amount of absorbent polymer material able to be deposited in the remaining storage cells, an overall decrease in liquid absorption and retention capacity arises. Furthermore the acquisition cells lead to higher rewet values on these specific locations since the liquid contained within these cells remains unbound in between the absorbent storage cells. This absorbent structure does thus not satisfactorily manage fluid absorption, distribution and retention and is therefore believed not to provide a satisfactory substantially cellulose free absorbent article.
EP2022452 discloses an absorbent article with an absorbent structure for the absorption of fluids comprising a non-woven supporting layer, a non-uniform layer of substantially cellulose free absorbent polymer material deposited thereon substantially continuously covered by a layer of a thermoplastic material (e.g. hot melt adhesive). Portions of the thermoplastic material are directly bonded through the absorbent polymer material layer to the supporting layer thereby defining thermoplastic pockets immobilizing the absorbent polymer material. While the thermoplastic pockets limit the migration and movement within the absorbent structure, the chemical gluing and physical restraining of the absorbent polymer material results in a significant reduction of liquid absorption, distribution and retention performance of the absorbent structure with decreased swelling and take up capacity of the absorbent polymer material. The thermoplastic pockets also giving rise to an uncomfortable stiff and rigid absorbent structure decreasing overall good fit and discreet wearing comport. While these substantially cellulose free absorbent articles thus comprise a thin absorbent structure with a relative high amounts of immobilized absorbent polymer material by way of a thermoplastic hot melt layer it is believed that this absorbent article does not provide satisfactory flexibility and comfort, nor does it provide optimal fluid management capacities since it leaves the available absorbent material greatly underused both in partially and fully liquid loaded state.

Whilst the above attempts describe various approaches to the above identified problems, it is believed that none of these concepts provides a very performing absorbent article. The absorbent materials left unused and the complex manufacturing processes makes neither of the above absorbent article economically, technically and/or environmentally advantageous.

Hence, there is still a need in the art for an improved thin, flexible, lightweight absorbent structure for use in an absorbent article which overcomes the problems of the prior art, essentially comprising a substantially cellulose free absorbent polymer material allowing effective and efficient management of available absorbent materials during absorption, distribution and retention of bodily exudates whilst providing a flexible, discreet fit and optimal wearing comfort. Furthermore, there is still a need for a method to produce the absorbent structures at a high and consistent production speed.

### SUMMARY OF THE INVENTION

As a result of exhaustive research to address the above-identified, derived and related problems, the inventors have found that excellent fluid management, fit and comfort can be achieved by substantially cellulose free absorbent structures, which will be explained in greater detail down below.

In particular, the present invention provides an absorbent structure for use in an absorbent article comprising:
a) a distribution layer having an absorbent capacity of at least about 5 g/m2;
b) an immobilisation layer which is joined to the distribution layer to define compartments there between; and
c) an absorbent material held in at least one of the compartments:
   i) wherein said absorbent material comprises an absorbent polymer material, and
   ii) from zero to an amount less than about 40 weight percent absorbent fibrous material, based on the weight of absorbent polymer material.

An absorbent structure according to an embodiment of the present invention is characterized by improved dispersion and transport properties of liquids from bodily exudates. The absorbent structure provided by the present invention is advantageous as it provides improved utilisation of absorbent materials when the absorbent article is partially or fully loaded with bodily exudates. Moreover the absorbent structure provided displays an increased product performance, for example excellent liquid-absorbing and retaining capabilities, high absorption speeds and low rewet values. The present invention furthermore provides an environmentally-friendly absorbent article which is capable of reducing the total amount of raw materials by increased efficiency and effectiveness of the raw materials used in the absorbent article.

In a further aspect, the invention provides an absorbing article comprising an absorbent structure as provided by the invention. In particular, the invention provides an absorbent article comprising a liquid pervious topsheet, a liquid impervious backsheet and a liquid absorbing absorbent structure according to an embodiment of the invention, situated in between the liquid pervious topsheet and liquid impervious backsheet. An absorbent article according to the invention has a discreet, improved fit and wearing comfort.

In another aspect the present invention provides a method for the manufacturing of an absorbent structure for use in an absorbent article comprising the steps of:
- providing a distribution layer having an absorbing capacity of at least about 5 g/m²_{;}
- covering the distribution layer with an absorbent material wherein said absorbent material comprises
   i) an absorbent polymer material, and
   ii) from zero to an amount less than about 40 weight percent absorbent fibrous material, based on the weight of absorbent polymer material;
- covering the absorbent material with an immobilisation layer which is joinable to the distribution layer; and
- joining the immobilisation layer to the distribution layer to define compartments there between wherein the absorbent material is held in at least one of the compartments.

Unlike previously existing substantially cellulose free absorbent articles and methods relating thereto limited by the relative concentrations of absorbent polymer material (e.g. gel blocking) to enable them to absorb liquid at the volume and rate at which the bodily exudates are applied to the absorbent article during use and with a rather stiff and rigid feel giving rise skin irritations and limited wearing comfort, the present invention overcomes the situation by well managed fluid handling, fit and comfort.

More specifically the invention provides increased fluid communication including adsorption and dispersion in and between the absorbent polymer material compartments, while additional wicking arises within the absorbent polymer material compartments which have until now been underused and unappreciated. Preferably the mass flow of liquids throughout the absorbent structure is also available.

The present invention thereby does not only provide for a significant increase in efficiency and effectiveness of raw materials available in the absorbent structure, it also limits gel blocking, reduces rewet and minimizes leakage.

Further advantageous features, aspects and details of the invention are evident from the dependent claims, the description and drawings as explained in more detail down below.

### BRIEF DESCRIPTION OF FIGURES

**Figure 1** provides a cross-sectional schematic illustration of an absorbent structure according to an embodiment of the invention.
**Figure 2** provides a cross-sectional schematic illustration of an absorbent structure according to another embodiment of the invention.
**Figure 3** provides a cross-sectional schematic illustration of an absorbent structure according to another embodiment of the invention.
**Figure 4** provides a top view schematic illustration of different area's of junction patterns of absorbent structures according to embodiments of the invention.
**Figure 5** provides a top view schematic illustration of different clusters of absorbent material of absorbent structures according to embodiments of the invention.
**Figure 6** is a top plan view of a diaper as a preferred embodiment of an absorbent article according to the invention, with the upper layers partially cut away.

### DETAILED DESCRIPTION OF THE INVENTION

The present invention concerns an improved absorbent structure for use in an absorbent article, preferably a disposable one, such as such as feminine hygiene garments, baby diapers and pants and adult incontinence garments; and to a method and manufacturing of the same.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

As used herein, the following terms have the following meanings:
"A", "an", and "the" as used herein refers to both singular and plural referents unless the context clearly dictates otherwise. By way of example, "a compartment" refers to one or more than one compartment.
"About" as used herein referring to a measurable value such as a parameter, an amount, a temporal duration, and the like, is meant to encompass variations of +/-20% or less, preferably +/-10% or less, more preferably +/-5% or less, even more preferably +/-1% or less, and still more preferably +/-0.1% or less of and from the specified value, insofar such variations are appropriate to perform in the disclosed invention. However, it is to be understood that the value to which the modifier "about" refers is itself also specifically disclosed.
"Absorbent article", "absorbent garment", "absorbent product", "absorbing article", "absorbing garment", "absorbing product" and the like as used herein are used interchangeably and refer to devices that absorb and contain bodily exudates, and more specifically, refers to devices that are placed against or in proximity to the body of the wearer to absorb and contain the various liquids discharged from the body. Absorbent articles include but are not limited to feminine hygiene garments, baby diapers and pants, adult incontinence garments, various diaper and pants holders, liners, towels, absorbent inserts and the like.
"Absorbent core" as used herein refers to a three-dimensional part of the absorbent structure, comprising liquid-absorbing material, useful to absorb and/or retain bodily exudates.
"Absorbent component" as used herein refers to a structural constituent of an absorbent structure, e.g., a piece of an absorbent core, such as one of multiple pieces in a multi-piece absorbent core.
"Absorbent element" as used herein refers to a part of a functional constituent of an absorbent structure, e.g., a liquid acquisition layer, a liquid distribution layer, or a liquid storage layer formed of a material or materials having particular liquid handling characteristics suitable for the specific function.
"Absorbent insert" as used herein refers to a device adapted for insertion into an absorbent article and to serve as an absorbent structure when so inserted.
"Absorbent layer" as used herein refers to a term referring to a discrete, identifiable sheet-like or web-like element of an absorbent structure which may remain detached and relatively movable with respect to another such element or may be attached or joined so as to remain permanently associated with another such element. Each absorbent layer may itself include a laminate or combination of several layers, sheets and/or webs of similar or diverse compositions.
"Absorbent polymer material", "absorbent gelling material", "AGM", "superabsorbent", "superabsorbent material", "super absorbent polymer", "SAP" and the like as used herein are used interchangeably and refer to any suitable particulate (e.g., flaked, particulate, granular, or powdered) or fibrous cross linked polymeric materials that can absorb at least 5 times and preferably at least about 10 times or more its weight of an aqueous 0.9% saline solution as measured using the Centrifuge Retention Capacity test (EDANA 441.2-01).
"Absorbent polymer material area" as used herein refers to the area of the absorbent structure wherein adjacent layers are separated by a multiplicity of absorbent polymer material. Incidental contact areas between these adjacent layers within the absorbent particulate polymer material area may be intentional (e.g. bond area's) or unintentional (e.g. manufacturing artefacts).
"Absorbent particulate polymer material" as used herein refers to an absorbent polymer material which is in particulate form such as powders, granules, flakes and the like so as to be flowable in the dry state.
"Absorbent structure" as used herein refers to those elements of an absorbent article comprising material or a combination of materials suitable to absorb, distribute and retain bodily exudates.
"Absorption" as used herein refers to the process by which a liquid is taken up within a material.
"Acquisition layer", "acquisition region", "acquisition surface" or "acquisition material" and the like as used herein refer to a layer having a faster liquid uptake capability.
"Absorbency" is the ability of a material to take up fluids by various means including capillary, osmotic, solvent, chemical or other action.
"Adult incontinence garment" as used herein refers to absorbent articles intended to be worn by incontinent adults, for absorbing and containing bodily exudates.
"Adhesion" as used herein refers to the force that holds different materials together at their interface.
"Adhesive" as used herein refers to a material, which may or may not be flowable in solution or when heated, that is used to bond materials together.
"Adsorption" as used herein refers to the process by which a liquid is taken up by the surface of a material.
"Air laying" as used herein refers to forming a web by dispersing fibres or particles in an air stream and condensing them from the air stream onto a moving screen by means of a pressure or vacuum; a web of fibres produced by air laying is herein referred to an "airlaid"; an airlaid web bonded by one or more techniques to provide fabric integrity is herein referred to an "airlaid nonwoven".
"Apparent density" as used herein refers to the basis weight of the sample divided by the calliper with appropriate unit conversions incorporated therein. Apparent density used herein has the unit g/cm³.
"Attach", "attached" and "attachment" as used herein are synonymous with their counterparts of the terms "fasten", "affix", "secure", "glue", "bind", "join" and "link".
"Baby diaper" as used herein refers to absorbent articles intended to be worn by children, for absorbing and containing bodily exudates which the user draws up between the legs and fastens about the waist of the wearer.
"Baby pants" as used herein refers to absorbent articles marketed for use in transitioning children from diapers to underwear intended to cover the lower torso of children, so as to absorb and contain body exudates which article is generally configured like a panty garment and manufactured with a completed waist encircling portion, thereby eliminating the need for the user to fasten the article about the waist of the wearer.
"Back region" as used herein refers to the portion of an absorbent article or part thereof that is intended to be positioned proximate the back of a wearer.
"Backing" as used herein refers to a web or other material that supports and reinforces the back of a product.
"Basis weight" is the weight per unit area of a sample reported in grams per square meter, g/m² or gsm.
"Bodily exudates", "body exudates", "bodily fluids", "body fluids", "bodily discharges", "body discharges", "liquids" and the like as used herein are used interchangeably and refer to, but are not limited to urine, blood, vaginal discharges, breast milk, sweats and faecal matter.
"Binder", "adhesive", "glue", "resins", "plastics" and the like as used herein are used interchangeably and refer to substances, generally in a solid form (e.g. powder, film, fibre) or as a foam, or in a liquid form (e.g. emulsion, dispersion, solution) used for example by way of impregnation, spraying, printing, foam application and the like used for attaching or bonding functional and/or structural components, elements and materials, for example including heat and/or pressure sensitive adhesives, hot-melts, heat activated adhesives, thermoplastic materials, chemical activated adhesives/solvents, curable materials and the like.
"Bond strength" as used herein refers to the amount of adhesion between bonded surfaces. It is a measure of the stress required to separate a layer of material from the base to which it is bonded.
"Capillary action", "capillarity", or "capillary motion" and the like as used herein are used to refer to the phenomena of the flow of liquid through porous media.
"Chassis" as used herein refers to a foundational constituent of an absorbent article upon which the remainder of the structure of the article is built up or overlaid, e.g., in a diaper, the structural elements that give the diaper the form of briefs or pants when configured for wearing, such as a backsheet, a topsheet, or a combination of a topsheet and a backsheet.
"Cellulose fibres" as used herein refers to naturally occurring fibres based on cellulose, such as, for example cotton, linen, etc; wood pulp fibres are one example of cellulose fibres; man-made fibres derived from cellulose, such as regenerated cellulose (rayon), or partially or fully acetylated cellulose derivatives (e.g. cellulose acetate or triacetate) are also considered as cellulose fibres.
"Cluster" or the like as used herein refers to an agglomeration of particles and/or fibres.
"Chemically stiffened fibres", chemically modified fibres", "chemically cross-linked fibres", "curly fibres" and the like as used herein are used interchangeably and refer to any fibres which have been stiffened by chemical means to increase stiffness of the fibres under both dry and aqueous conditions, for example by way of addition of chemical stiffening agents (e.g. by coating, impregnating, etc), altering the chemical structure of the fibres themselves (e.g. by cross-linking polymer chains, etc) and the like.
"Cohesion" as used herein refers to the resistance of similar materials to be separated from each other.
"Compartment" as used herein refers to chambers, cavities, pockets and the like.
"Comprise," "comprising," and "comprises" and "comprised of" as used herein are synonymous with "include", "including", "includes" or "contain", "containing", "contains" and are inclusive or open-ended terms that specify the presence of what follows e.g. a component and do not exclude or preclude the presence of additional, non-recited components, features, elements, members, steps, known in the art or disclosed therein.
"Coverstock" as used herein refers to a lightweight non-woven material used to contain and conceal an underlying absorbent core material; examples are the facing layer or materials that cover the absorbent cores of feminine hygiene garments, baby diapers and pants and adult incontinence garments.
"Crotch region" of an absorbent article as used herein refers to about 50% of the absorbent article's total length (i.e., in the y-dimension), where the crotch point is located in the longitudinal centre of the crotch region. That is, the crotch region is determined by first locating the crotch point of the absorbent article, and then measuring forward and backward a distance of 25% of the absorbent article's total length.
"Cross direction (CD)", "lateral" or "transverse" and the like as used herein are used interchangeably and refer to a direction which is orthogonal to the longitudinal direction and includes directions within ±45° of the transversal direction.
"Curing" as used herein refers to a process by which resins, binders or plastics are set into or onto fabrics, usually by heating, to cause them to stay in place; the setting may occur by removing solvent or by cross-linking so as to make them insoluble.
"Diaper", "conventional diaper", "diaper-like", "diaper-like garment" and the like as used herein are used interchangeably and refer to disposable absorbent articles, which typically include a front waist portion and a back waist portion which may be releasably connected about the hips of the wearer during use by conventional fasteners such as adhesive tape fasteners or hook and loop type fasteners. In use, the article is positioned between the legs of the wearer and the fasteners are releasably attached to secure the back waist portion to the front waist portion of the diaper, thereby securing the diaper about the waist of the wearer. The front waist portion and a back waist portion are connected by relatively non-stretchable or stretchable members (the term "stretchable" as used herein refers to materials that are extensible when forces are applied to the material, and offer some resistance to extension). Hence, such articles are generally not configured to be pulled up or down over the hips of the wearer when the fasteners are attached.
"Disposable" is used herein to describe articles that are generally not intended to be laundered or otherwise restored or reused (i.e., they are intended to be discarded after a single use and, preferably, to be recycled, composted or otherwise disposed of in an environmentally compatible manner).
"Distribution layer", "distribution region", "distribution surface" or "distribution material" and the like as used herein are used interchangeably and refer to a layer having a larger capacity in wicking, dispersing and distributing liquids.
"Dry laying" as used herein refers to a process for making a nonwoven web from dry fibre; these terms apply to the formation of carded webs, as well as to the air laying formation of random webs; a web of fibres produced by dry laying is herein referred to as a "drylaid"; a drylaid web bonded by one or more techniques to provide fabric integrity is herein referred to a "drylaid nonwoven".
"Dry strength" as used herein refers to the strength of an adhesive joint determined in dry state conditions, immediately after drying under specified conditions or after a period of conditioning in the standard laboratory atmosphere.
"Fabric" as used herein refers to a sheet structure made from fibres, filaments and/or yarns.
"Feminine hygiene garments" as used herein refer to absorbent hygiene articles intended to be worn by woman, for absorbing and containing body exudates.
"Fibre" as used herein refers to the basic threadlike structure from which nonwovens, yarns and textiles are made. It differs from a particle by having a length at least 4 times its width; "Natural fibres" are either of animal (wool, silk), vegetable (cotton, flax, jute) or mineral (asbestos) origin, while "Man-made fibres" may be either polymers synthesised from chemical compounds (polyester, polypropylene, nylon, acrylic etc.) or modified natural polymers (rayon, acetate) or mineral (glass). "Fibre" and "filament" are used interchangeably.
"Film", "foil" and the like as used herein are used interchangeably and refer to a thin sheet of essentially non-absorbent material such as plastic or closed foams. In this invention it particularly refers to materials that do not correspond to non-woven.
"Fluff pulp" as used herein refers to wood pulp specially prepared to be dry laid.
"Front region" as used herein refers to the portion of an absorbent article or part thereof that is intended to be positioned proximate the front of a wearer.
"Garment facing layer" as used herein refers to elements of the chassis that form the outer surface of the absorbent article, such as the back sheet, the side panels, the waist fasteners, and the like, when such elements are present.
"Heat activated adhesive" as used herein refers to a dry adhesive that is rendered tacky or fluid by application of heat or heat and pressure to the assembly.
"Heat sealing adhesive" as used herein refers to a thermoplastic adhesive which is melted between the adherent surfaces by heat application to one or both of the adjacent adherent surfaces.
"Highloft" as used herein refers to general term of low density, thick or bulky fabrics.
"Hot-melt adhesive" as used herein refers to a solid material that melts quickly upon heating, then sets to a firm bond upon cooling; used for almost instantaneous bonding.
"Hydrophilic" as used herein refers to having an affinity for being wetted by water or for absorbing water.
"Hydrophobic" as used herein refers to lacking the affinity for being wetted by water or for absorbing water.
"Immobilisation layer" as used herein refers to a layer able to be applied to the absorbent polymer material with the intent to bond and/or immobilize absorbent material.
"Join", "joined" and "joining" as used herein refers to encompassing configurations wherein an element is directly secured to another element by affixing the element directly to the other element, as well as configurations wherein the element is indirectly secured to the other element by affixing the element to an intermediate member or members which in turn is or are affixed to the other element.
"Knitting" as used herein refers to the technique for interlocking loops of fibres with needles or similar devices.
"Layer" refers to identifiable components of the absorbent article, and any part referred to as a "layer" may actually comprise a laminate or combination of several sheets or webs of the requisite type of materials. As used herein, the term "layer" includes the terms "layers" and "layered." "Upper" refers to the layer of the absorbent article which is nearest to and faces the wearer facing layer; conversely, the term "lower" refers to the layer of the absorbent article which is nearest to and faces the garment facing layer. "Layer" is three dimensional structure with a x dimension width, y dimension length, and z-dimensions thickness or calliper, said x-y dimensions being substantially in the plane of the article, however it should be noted that the various members, layers, and structures of absorbent articles according to the present invention may or may not be generally planar in nature, and may be shaped or profiled in any desired configuration.
"Machine direction (MD)", "longitudinal" and the like as used herein are used interchangeably and refer to a direction running parallel to the maximum linear dimension of the structure and includes directions within ±45° of the longitudinal direction.
"Major surface" as used herein refers to a term used to describe the surfaces of greatest extent of a generally planar or sheet-like structural element and to distinguish these surfaces from the minor surfaces of the end edges and the side edges, i.e., in an element having a length, a width, and a thickness, the thickness being the smallest of the three dimensions, the major surfaces are those defined by the length and the width and thus having the greatest extent.
"Mass flow" as used herein refers to the flow of a liquid from one absorbent element or component to another absorbent element or component by channel flow action.
"Mechanical bonding" as used herein refers to a method of bonding fibres by entangling them. This can be achieved by needling, stitching with fibres or by the use of high-pressure air or water jets and the like.
"Non-woven" as used herein refers to manufactured sheet, web or batt of directionally or randomly orientated fibres, bonded by friction, and/or cohesion and/or adhesion, excluding paper and products which are woven, knitted, tufted, stitch-bonded incorporating binding yarns or filaments, or felted by wet-milling, whether or not additionally needled. The fibres may be of natural or man-made origin and may be staple or continuous filaments or be formed in situ. Commercially available fibres have diameters ranging from less than about 0.001 mm to more than about 0.2 mm and they come in several different forms: short fibres (known as staple, or chopped), continuous single fibres (filaments or monofilaments), untwisted bundles of continuous filaments (tow), and twisted bundles of continuous filaments (yarn). Nonwoven fabrics can be formed by many processes such as melt blowing, spun bonding, solvent spinning, electro spinning, and carding. The basis weight of nonwoven fabrics is usually expressed in grams per square meter (gsm).
"Pant", "training pant", "closed diapers", "prefastened diapers", "pull-on diapers" and "diaper-pants" and the like as used herein are used interchangeably and refer to absorbent articles which are typically applied to the wearer by first leading the feet into the respective leg openings and subsequently pulling the pants from the feet to waist area over the hips and buttocks of the wearer and which are capable of being pulled up or down over the hips of the wearer. Typically, such articles may include a front waist portion and a back waist portion which may be connected about the hips of the wearer by integral or releasable members. A pant may be preformed by any suitable technique including, but not limited to, joining together portions of the article using refastenable and/or non-refastenable bonds (e.g., seam, weld, adhesive, cohesive bond, fastener, etc.). A pant may be preformed anywhere along the circumference of the article (e.g., side fastened, front waist fastened).
"Polymer" as used herein refers to but is not limited to, homopolymers, copolymers, such as for example, block, graft, random and alternating copolymers, terpolymers, etc. and blends and modifications thereof. Unless otherwise specifically limited, the term "polymer" includes all possible spatial configurations of the molecule and include, but are not limited to isotactic, syndiotactic and random symmetries.
"Rear" as used herein refers to the portion of an absorbent article or part thereof that is intended to be positioned proximate the back of the wearer.
"Resin" as used herein refers to a solid or semisolid polymeric material.
"Substantially cellulose free" as used herein refers to an absorbent article, structure or core, that contains less than 40% by weight cellulosic fibres, less than 20% cellulosic fibres, less than 5% cellulosic fibres, no cellulosic fibres, or no more than an immaterial amount of cellulosic fibres which do not materially affect the thinness, flexibility or absorbency thereof.
"Thermobonding" as used herein refers to a method of bonding fibres by the use of heat and/or high-pressure.
"Thermoplastic" as used herein refers to polymeric materials that have a melting temperature and can flow or be formed into desired shapes on the application of heat at or below the melting point.
"Ultrasonic" as used herein refers to the use of high frequency sound to generate localised heat through vibration thereby causing thermoplastic fibres to bond to one another.
"Water-absorbing", "liquid-absorbing", "absorbent", "absorbing" and the like as used herein are used interchangeably and refer to compounds, materials, products that absorb at least water, but typically also other aqueous fluids and typically other parts of bodily exudates such as at least urine or blood.
"Wearer facing layer" as used herein refers to elements of the chassis that form the inner surface of the absorbent article, such as the topsheet, the leg cuffs, and the side panels, etc., when such elements are present.
"Weaving" as used herein refers to the process of interlacing two or more sets of yarns at right angles to form a fabric; a web of fibres produced by weaving is herein referred to as a "Woven".
"Web material" as used herein refers to an essentially endless material in one direction, i.e. the longitudinal extension or the length, or the x- direction in Cartesian coordinates relative to the web material. Included in this term is an essentially unlimited sequence of pieces cut or otherwise separated from an essentially endless material. Often, though not necessarily, the web materials will have a thickness dimension (i.e. the z-direction) which is significantly smaller than the longitudinal extension (i.e. in x-direction). Typically, the width of web materials (the y-direction) will be significantly larger than the thickness, but less than the length. Often, though not necessarily, the thickness and the width of such materials is essentially constant along the length of the web. Without intending any limitation, such web materials may be cellulosic fiber materials, tissues, woven or non-woven materials and the like. Typically, though not necessarily, web materials are supplied in roll form, or on spools, or in a folded state in boxes. The individual deliveries may then be spliced together to form the essentially endless structure. A web material may be composed of several web materials, such as multilayer non-woven, coated tissues, non woven / film laminates. Web materials may comprise other materials, such as added binding material, particles, hydrophilizing agents and the like.
"Wet burst strength" is a measure of a layer's ability to absorb energy, when wet and subjected to deformation normal to the plane of the web.
"Wet strength" as used herein refers to the strength of an adhesive joint determined immediately after removal from a liquid in which it has been immersed under specified conditions of time, temperature and pressure. The term is commonly used in the art to designate strength after immersion in water.
"Wet laying" as used herein refers to the forming a web from an aqueous dispersion of fibres by applying modified paper making techniques; a web of fibres produced by wetlaying is herein referred to as a "wetlaid".
"Wood pulp" as used herein refers to cellulosic fibres used to make viscose rayon, paper and the absorbent cores of products such as feminine hygiene garments, baby diapers and pants and adult incontinence garments.
"X-y dimension" as used herein refers to the plane orthogonal to the thickness of the article, structure or element. The x- and y-dimensions correspond generally to the width and length, respectively, of the article, structure or element.
"Z-dimension" as used herein refers to the dimension orthogonal to the length and width of the article, structure or element. The z-dimension corresponds generally to the thickness of the article, structure or element.

The recitation of numerical ranges by endpoints includes all numbers and fractions subsumed within that range, as well as the recited endpoints.

The present invention has resulted from a realization that an absorbent article having a liquid-permeable top sheet, a liquid-impermeable back sheet, and an absorbent structure, wherein the absorbent structure comprises a distribution layer, an immobilisation layer joined to the distribution layer to define compartments there between and wherein absorbent material is held in at least one of the compartments, can be more efficiently used to absorb, distribute and retain liquids, such as urine, if the absorbent structure is provided with a distribution layer having a capability to disperse the liquid permeating within said distribution layer from the less absorbent area's (e.g. saturated) to the more absorbent area's (e.g. unsaturated).

In particular, the present invention provides an absorbent structure for use in an absorbent article comprising:
a) a distribution layer having an absorbent capacity of at least about 5 g/m2;
b) an immobilisation layer which is joined to the distribution layer to define compartments there between; and
c) an absorbent material held in at least one of the compartments:
   i) wherein said absorbent material comprises an absorbent polymer material, and
   ii) from zero to an amount less than about 40 weight percent absorbent fibrous material, based on the weight of absorbent polymer material.

In the present invention the term "distribution layer" refers to the layer substantially or essentially in contact with the surface of the absorbent material layer (e.g. absorbent polymer material area) not facing the immobilisation layer. Although preferably the distribution layer is located at the garment facing side and the immobilisation layer is preferably located at the wearer facing side of the absorbent polymer material area, it is understood that the orientation of the absorbent structure, although not preferred, can also be re-orientated so as to locate the distribution layer at the wearer facing side and the immobilisation layer at the garment facing side of the absorbent polymer material area, while various combinations and variations are of course also possible. Due to the specific absorbent capacity of the distribution layer, the liquid around the bonds in between the compartments will be drawn up into the distribution layer and will spread out throughout the rest of the distribution layer. In a preferred embodiment of the invention, the liquid wetting the distribution layer will therefore also contact the lower side of the absorbent polymer material area's typically fully or partially closed of by the upper immobilisation layer and the upper gel-formation and/or -blocking. The absorbent material held in the compartments will thus advantageously be contacted from the lower layer by redirecting the liquid from the upper layer through the distribution layer into the absorbent material, such as absorbent polymer material.

In a preferred embodiment the distribution layer has an absorbing capacity of at least about 1, 2, 3, 4 or 5 g/m², preferably about 10 g/m², more preferably about 20 g/m², more preferably at least about 35 g/m², most preferably at least about 50 g/m², or 100 g/m². Preferably it is presented in the form of a substantially continuous sheet of cellulosic fibers, such as a sheet of paper or tissue.

The absorbent capacity of the distribution layer can be measured by techniques known to a person skilled in the art,

In a preferred embodiment of the absorbent article according to an embodiment of the invention, the distribution layer is provided by a substantially continuous layer comprising absorbent cellulosic fibres, preferably in the form of paper or tissue.

In the present invention the term "substantially continuous layer" refers to materials presented in the form of a sheet, such as for instance paper or tissue. This reference includes sheets with a high degree of porosity, with perforations or openings, but is meant to exclude non-woven. Such paper and/or tissues can for instance, but not exclusively, be made by the various techniques know in the art, such as for instance air laying and wet laying processes.

In the present invention the term "absorbent cellulosic fibres" refers to cellulose fibres with hydrophilic properties. In the present invention the term "paper" refers to a material made of cellulose pulp, derived mainly from wood, rags, and certain grasses, processed into flexible sheets or rolls by deposit from an aqueous suspension or as a result of a dry laying or air laying process.

In the present invention the term "tissue" refers to an absorbent piece of paper.

In a preferred embodiment, the substantially continuous layer has a basis weight range where the low limit of the range is about 1, 2, 3, 4, 5, 6, 7, 8, 9, 10 g/m² per ply, about 13 g/m² per ply, or about 15 g/m² per ply. The high limit of the basis weight range is about 150 g/m² per ply, 100 g/m² per ply, about 40 g/m² per ply, or about 25 g/m² per ply. Ranges in between are hereby also included in the preferred embodiments.

Generally, identical plies of the substantially continuous layer are used, that is plies, substantially identical in basis weight, thickness, composition and other properties. However, it is contemplated that certain benefits may be realized by using plies having differing properties. For example, the component plies may differ in basis weight, thickness, composition or other properties, providing one side of the substantially continuous layer with a relatively highly absorbent surface, and one side with a relatively little absorbent surface.

The plies of the tissue can be passively bound or joined, or a certain amount of adhesive or other attachment means could be added to provide additional adhesion to portions of the component plies. For example, needling, embossing, or other thermal or mechanical bonding means could be used to actively bond the substantially continuous layer near some or all of the edges of the sheet, thereby providing increased resistance to undesired delaminating of the component plies.

Joining may also be by ultrasonic bonding or autogenous bonding, or other bonding methods known in the art. For example, if the edges of the ply or layers are coextensive with the edges of the outer plies, adhesive bonding and heat sealing adhesive may not provide active bonding, depending on the adhesive used and the surface energy characteristics of the ply. In this case, mechanical bonding may be more desirable, for example by mechanical bonding at a mechanical bonding station after formation of the multiple ply web. Depending on the structural and functional requirements the bonding parameters may be adjusted so as to obtain the most suitable dry and wet strength values.

If desired, multiple plies of the substantially continuous layer may be joined and embossed. If desired, the plies may be joined together using knob-to-knob or know-to-flat surface embossing as is known in the art. Alternatively, the multiple plies may be embossed using nested embossing.

The substantially continuous layer manifests various physical characteristics. In a preferred embodiment, the distribution layer has an absorbency ranging from about 0.1 g to 3 g water per g of distribution layer, preferably from about 3 to 7 g, more preferably from about 7 to 10 g and preferably the absorbency is higher than about 10 g water per g of distribution layer. Despite the high absorbency and distribution capacities of the substantially continuous layer, the bulkiness of said layer is absolutely and relatively limited, for instance in comparison with absorbent articles and absorbent structures from the art allowing the envisaged thinner, more flexible and comfortable structures.

Basis weight and apparent density relate to the bulkiness of the layer. For the present invention, the substantially continuous layer can have a basis weight ranging from about 5 g/m² to about 150 g/m². Preferably, the substantially continuous layer can have a basis weight ranging from about 13 g/m² to about 23 g/m². More preferably, the substantially continuous layer can have a basis weight of about 16-18 g/m².

The substantially continuous layers preferably have sufficient strength to perform their intended tasks. Preferably, the substantially continuous layer maintains its integrity when partially or fully wetted, so that the distribution of liquids to and the immobilization of the absorbent materials may be accomplished. The distribution layer preferably has a wet burst strength ranging from a lower limit of about 50-75 g and preferably about 200 g, to an upper limit of about 800 g, more preferably about 600 g, and most preferably about 400 g. This is advantageous as it provides the layer with a good combination of absorbency and wet strength.

The parameters wet burst strength, basis weight, absorbent capacity, and absorbent capacity, can be determined by analytical methods known to a person skilled in the art, for instance as described in US 6162327, herein incorporated by reference.

The substantially continuous layer can be creped, uncreped, or wet micro-contracted tissue webs. The substantially continuous layer may be paper or tissue consisting essentially of cellulosic papermaking fibers. Optionally, the substantially continuous layer may be foreshortened, and/or contain synthetic fibers.

Preferably, the substantially continuous layer according to the present invention is blended. By being blended, it is meant that the layer comprises a homogeneous mixture of papermaking fibers. The homogeneous mixture preferably comprises both softwood and hardwood fibers. The fibers may be refined using any commercially available process and may include recycled fibers.

The immobilisation layer according to the invention can be any material suitable to keep the absorbent materials within the compartments in their intended place when bonded to the distribution layer, such as for instance a non-woven material, paper or tissue. In a preferred embodiment of the absorbent article of the invention, the immobilisation layer is a thermoplastic adhesive. Use of a thermoplastic adhesive as immobilisation layer is advantageous as the material is easily bondable to a substantially continuous layer, in particular in the form of paper or tissue. The major surface of the absorbent material, preferably comprising absorbent polymer materials, more preferably absorbent particulate polymer material, is preferably located in between the immobilisation layer and the distribution layers to maximise the effectiveness and efficiency of the invention.

In a preferred embodiment of the absorbent article of the invention, the absorbent polymer material is present in the absorbent material in an average basis weight of at least about 100 g/m², preferably at least about 200 g/m², more preferably at least about 400 g/m², even more preferably at least about 600 g/m², most preferably at least 700 g/m². Use of a absorbent polymer material as absorbent material provides an improved absorption capacity whereby the relative high density leads to the flexible and less bulky absorbent articles.

In a preferred embodiment of the absorbent article of the invention, the absorbent material is a super absorbent polymer (SAP). Preferably, the super absorbent polymer is present in the absorbent material in an average basis weight of at least about 100 g/m², preferably at least about 200 g/m², more preferably at least about 400 g/m², even more preferably at least about 600 g/m², most preferably at least about 700 g/m². Use of super absorbent polymer material as absorbent material provides an improved absorption capacity whereby the relative high density leads to the flexible and less bulky absorbent articles.

In a preferred embodiment of the absorbent article according to the invention, the bonds, joints and connections between the garment facing surface of the immobilizing layer and the wearer facing surface of the distribution medium are not permanent so that the bonds may partially break, detach and/or disintegrate during wetting.

The absorbent structure of the present invention has a low flexure-resistance. The flexure-resistance of the absorbent structures is measured by the Peak Bending Stiffness as determined by the test which is modelled after the ASTM D 4032-82 Circular Bend Procedure, the procedure being considerably modified and performed as described in EP0336578B1, hereby incorporated by reference. In a preferred embodiment of the absorbent article of the invention, the absorbent structure has a flexure-resistance of less than about 500.0 grams, more preferably less than about 250.0-350.0 grams, and still more preferably less than about 175.0 grams and most preferably less than about 130.0 or 100.0 grams. Thus, the absorbent structure of the present invention is highly flexible and conforms very well to the various shapes of the urogenital region. Use of an absorbent structure with this low a flexure-resistance has for effect that an easily flexible discreet structure can be provided. This feature is advantageous as such a structure will easily ply and allow the structure to follow a body shape and will as such not be perceived as rigid, uncomfortable, irritating, bulky or noisy. Consequently, improved wearer fit and comfort is provided.

The absorbent structure of the present invention has minimum wet immobilisation values as measured by the shaker test weight retention as determined by the Wet Immobilisation test as described in US Patent Application 20070167928, hereby incorporated by reference. In a preferred embodiment of the absorbent article of the invention, the absorbent structure has a wet immobilization of more than about 50 wt%, preferably more than about 70 wt%, more preferably more than about 80 wt%, and most preferably more than about 90 wt%. An absorbent structure according to an embodiment of the invention has an increased loading capacity and an improved absorption capacity is available.

In a second aspect, the present invention provides an absorbent article comprising, at least in the front half of the absorbent article, an absorbent structure according to an embodiment of the invention. It is known that for most absorbent articles, for instance for articles such as feminine hygiene garments, baby diapers, baby pants and adult incontinence products, liquid discharge occurs predominantly in the front half. It is therefore advantageous to provide an improved absorbent article according to an embodiment of the invention in that area where fluid uptake requirement is highest. Obviously, an absorbent article comprising an absorbent structure according to an embodiment of the invention which is entirely or partially located in either the front, crotch and/or back region of the absorbent article, such as for instance a diaper, is also covered under this invention.

In a preferred embodiment, the absorbent article comprises an absorbent structure comprising a distribution layer having an upper distribution surface facing the wearer's skin and a lower distribution surface facing the garment of the wearer. The distribution layer is preferably in direct contact with the lower surface of the absorbent material, the absorbent core or the lower core wrap material. The distribution layer typically has an absorbent capacity of at least about 5-10 g/m², preferably at least about 30 g/m², more preferably at least about 50 g/m², more preferably at least about 75 g/cm², preferably at least about 100 g/cm² or 150 g/cm². Preferably it is presented in the form of a substantially continuous sheet of cellulosic fiber, such as a sheet of paper, tissue or a drylaid, airlaid or wetlaid material.

In a preferred embodiment, the absorbent article has a crotch width of less than about 90 mm. An absorbent article with a crotch width of this dimension provides improved wearer comfort. A preferred article according to the invention achieves a crotch width of less than about 85 mm, 80 mm, 75 mm, 70 mm, 65 mm, 60 mm, 55 mm or 50 mm. Hence, preferably an absorbent structure according to the present invention has a crotch width as measured along a transversal line with which is positioned at equal distance to the front edge and the rear edge of the core which is of less than about 100 mm, 90 mm, 80 mm, 70 mm, 60 mm or even less than 50 mm.

In a preferred embodiment of the invention, the absorbent structure is provided with a cover layer comprising a non-woven material.

In a preferred embodiment of the invention, portions of the cover layer bond or join to portions of the distribution layer via the immobilisation material; the distribution layer together with the immobilisation layer and cover layer providing cavities for the immobilization of the absorbent material, preferably absorbent polymer material. The bonding strength should suffice the required parameters of the absorbent structure and will amongst others depend on the product size, shape, category and required and duration of the usage and performance.

In a preferred embodiment of the absorbent article of the invention, the upper and lower layers of the absorbent structure are attached to each other at both the front and back ends of the absorbent core.

In a preferred embodiment of the invention, the portions of the cover layer bonded to the portions of the distribution layer provide areas of junction, said areas of junction are arranged along lines or points parallel with the longitudinal or transversal lines. More preferably these lines or points form an angle of at least about 5-40 degrees, more preferably 10 to 30 degrees with the longitudinal edges of the absorbent structure. The effect provided by the slightly inclined areas of junction is that of a ply layer. It allows that the form of the absorbing article better matches that of the body of a subject. It provides more flexibility to the structure. Consequently improved comfort can be obtained.

In a third aspect, the invention provides a method for the manufacturing of absorbent structures according to an embodiment of the invention. In particular, the invention provides a method for the manufacturing of an absorbent structure for use in an absorbent article comprising the steps of:
- providing a distribution layer having an absorbing capacity of at least about 5 g/m2;
- covering the distribution layer with an absorbent material wherein said absorbent material comprises
   i) an absorbent polymer material, and
   ii) from zero to an amount less than about 40 weight percent absorbent fibrous material, based on the weight of absorbent polymer material;
- covering the absorbent material with an immobilisation layer which is joinable to the distribution layer; and
- joining the immobilisation layer to the distribution layer to define compartments there between wherein the absorbent material is held in at least one of the compartments.

The process for producing preferred absorbent structures in accordance with the present invention may be as follows. The absorbent structure is laid down onto a down drum, which presents a perforated surface containing openings in line with the preferred locations of the absorbent polymer materials. In a first process step the distribution layer is laid on to the perforated surface and the absorbent polymer material is disposed by means know in the art. By using a vacuum means the absorbent polymer material will be drawn within the contours of the perforated vacuum means and thereby the absorbent polymer material will accumulate and cluster in predetermined locations according to the predefined perforation shapes. In a further process step an immobilisation layer is placed onto the absorbent polymer material. This immobilisation layer can be provided in the form of any suitable layer which can relatively immobilize and/or restrain the absorbent polymer materials once bound to the distribution layer, the immobilisation layer ranging for example from any sort of fabric, non-woven, paper, tissues to glue and binders. In case of a non inherent adhesive immobilisation layer, the immobilisation layer will need to be joined to the distribution layer to obtain the compartments containing the clusters of absorbent material. If an inherent adhesive immobilisation layer, either with or without activation, is used the immobilisation will be automatically joined to the distribution layer to obtain the compartments containing the clusters of absorbent material, although functional or complementary bonding by any means of the art (e.g. sonic bonding, adhesive, heat, pressure, etc) can be used to obtain the desired bonding strength along these area's of junctions. While any immobilisation application means known in the art can be used to place the immobilisation layer onto the absorbent polymer material, in case of for instance a hot melt adhesive, the binder is preferably applied by a nozzle system. Preferably, a nozzle system which can provide a relatively thin but wide curtain of binder is utilised. This glue curtain can be continuous or discontinuous, so as to be applied in a homogeneous or heterogeneous surface or can be applied in various combinations of lines, grids, spirals, figures, spots, dots, etc. either in a determined or undetermined location of the target surface and/or any combination thereof. This curtain of adhesive may be placed onto the distribution layer and/or the absorbent polymer material so as to immobilise and/or compartmentalize the absorbent material. Various types of glues and binders can be used within the absorbent structure, this does not necessarily need to one and the same type. As the openings on the distribution or wicking network are less covered by absorbent polymer material the binder or immobilisation area's of junction will make contact with these areas of the distribution layer and thereby capturing and immobilising the absorbent polymer materials. Various combinations and derivations are encompassed.

In an optional further process step a cover layer is placed upon the distribution layer, the absorbent polymer material and the immobilisation layer, for instance a hot melt adhesive layer. The cover layer will be bonded with the distribution layer in the areas of junction by any means know in the art, without limitation for instance adhesives, sonic boding, heat and/or pressure bonding, etc. In these areas of junction there is direct or indirect contact with the distribution layer. The cover layer will typically not be in adhesive contact with the distribution layer where the valleys of the distribution layer are filled with absorbent polymer material.

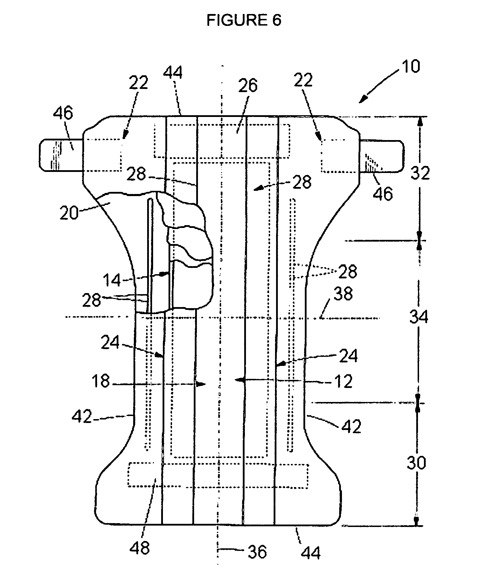

Alternatively the cover layer can be laid down onto a drum and the distribution network can be added in a consecutive process step. The embodiment shown in Fig. 6 could be produced by such a process.

In one alternative embodiment, the cover layer and the distribution layer are provided from a unitary sheet of material. The placing of the cover layer onto the distribution layer will then involve the folding of the unitary piece of material.

Hence, the uneven service of the lay-down system, which preferably is a lay-down drum, typically determines the distribution of absorbent polymer material throughout the absorbent material layer and likewise determines the pattern of areas of junction. Alternatively, the distribution of absorbent polymer material may be influenced by vacuum, air jet, conveyer surface, gravity, electrical or other means.

In a fourth aspect, an absorbing structure according to an embodiment of the invention is used as an absorbent core in a feminine hygiene garment, baby diaper, baby pants, or adult incontinence garment product.

While particular embodiments of the present invention have been illustrated and described, it would be obvious to those skilled in the art that various other changes and modifications can be made without departing from the spirit and scope of the invention.

Examples are used below for further non-limitative illustration of the invention.

### Example 1: Preparation of an absorbent structure according to an embodiment of the invention

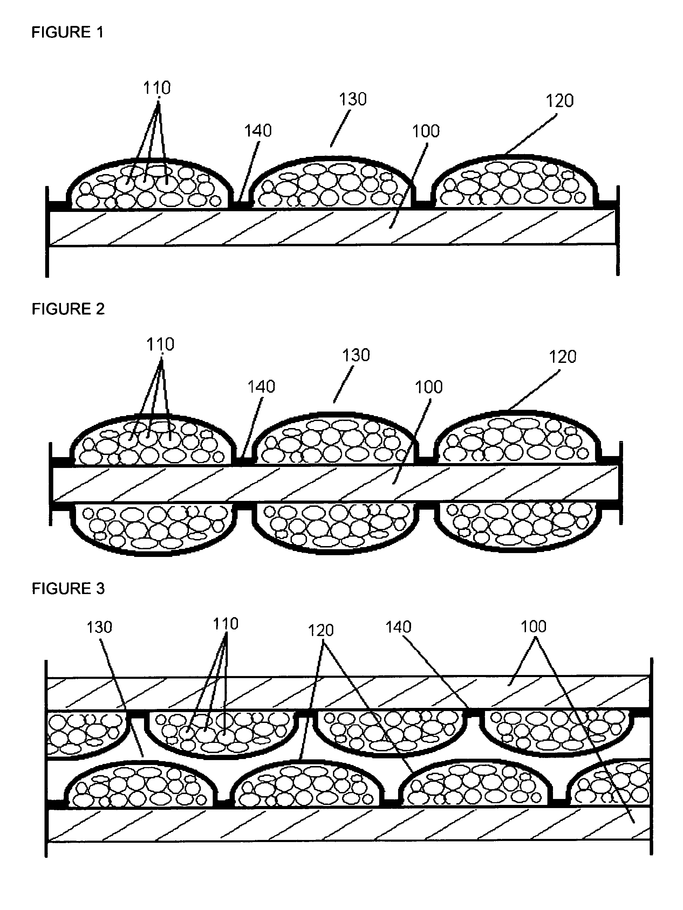

Referring to Figure 1, a distribution layer 100 having an absorbent capacity of at least 30 g/m2 is provided. The distribution layer 100 is covered on at least one side by discrete amounts of absorbent material 110. The absorbent material 110 is covered by an immobilisation layer 120. The immobilisation layer 120 lies on top of the absorbent material 110 and is bonded at regular intervals by area's of junction 140 to the distribution layer 100 thereby providing compartments 130 holding the absorbent material 110. The absorbent material 110 preferably comprises super absorbent polymer particles. In addition, absorbent fibrous materials can be used. Preferably the amount of absorbent fibrous material used is less than 20 weight percent based on the total weight of absorbent polymer material present.

Referring to Figure 2, it has been found that absorbent structures 14 can be formed by combining two layers of absorbent material 110. The absorbent structure 14 as shown in Figure 2 comprises one distribution layer 100, two layers of absorbent material 110 and two immobilisation layers 120. When two discontinuous layers of an absorbent material 110 are used, being one on the wearer facing surface and one on the garment facing surface, they would be typically arranged in such a way that the compartments 130 containing the absorbent material 110 from one storage layer are aligned with the compartments 130 containing the absorbent material 110 from the other storage layer in order to have the area's of junction 140 from both layers adjacent to one another. In another alternative embodiment, however, the compartments 130 and the respective area's of junction 140 are offset one another.

Referring to Figure 3, it has also been found that absorbent structures 14 can be formed by combining two or more layers of absorbent material 110. The absorbent structure 14 as shown in Figure 3 comprises two distribution layers 100, two layer of absorbent material 110 and two immobilisation layers 120. When two discontinuous layers of an absorbent material 110 are used, they would typically be arranged in such a way that the compartments 130 containing the absorbent material 110 of one storage layer faces the compartments 130 containing the absorbent material 110 of the other storage layer. In an alternative preferred embodiment, however, the area's of junction 140 are offset and do not face each other. Hence preferably, when two storage layers are joined, this is done such that the first immobilisation layer 120 of the first storage layer faces the immobilisation layer 120 of the second storage layer, while the two distribution layers 100 from each storage layer is situated on the wearer facing surface and the garment facing surface of the sandwiched absorbent structure 14.

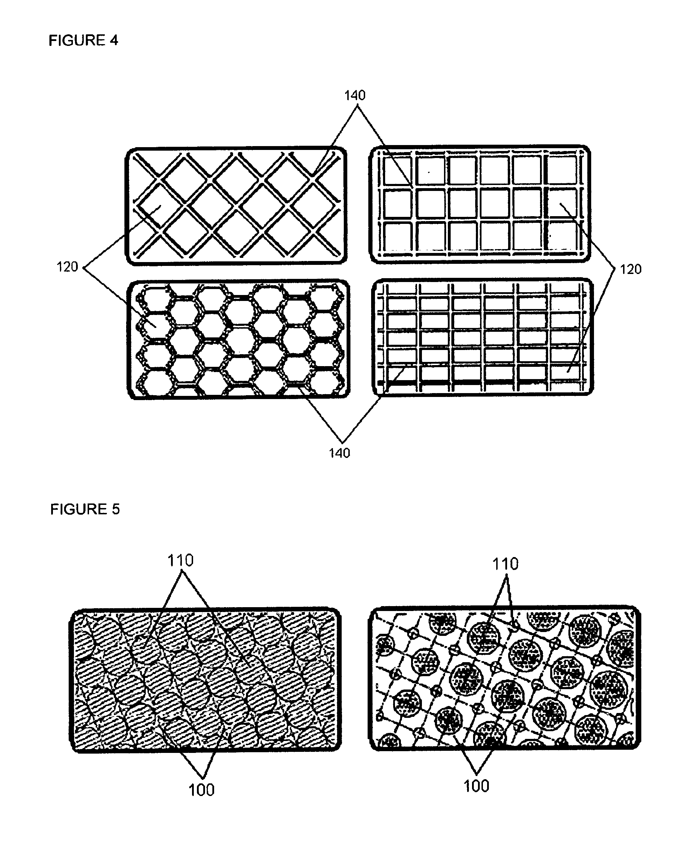

Referring to Figure 4, the areas of junction 140 can be disposed in different patterns. The lines of these patterns may be disposed regular or irregular, continuous or discontinuous, coextensive with the entire surface of the absorbent structure or only part thereof and any combination and/or derivations thereof. These lines may be aligned with the longitudinal, transversal or diagonal axis of the absorbent structure 14 or alternatively any angle in respect of such axis. It has been found that, that a continuous disposition of these lines throughout the absorbent structure 14 creates channels and ducts which help the fluid transport from the point of liquid contact to the rest of the absorbent structure via mass flow allowing better and more spread out immediate fluid management via capillary flow locally. Another preferred pattern for areas of junction 140 is a pattern comprising polygons, for example pentagons and sexangles or a combination thereof. Also preferred are irregular patterns of area's of junction 140, which have been found to give good wet immobilisation of the absorbent material 110.

Referring to Figure 5, the clusters of absorbent material 110 can be disposed in different patterns, sizes and locations. The disposition may be regular or irregular, continuous or discontinuous, covering the entire surface of the absorbent structure or only part thereof and any combination and derivations thereof.

### Example 2: Preparation of an absorbent article according to an embodiment of the invention

Figure 6 is a top plan view of a diaper 10 as a preferred embodiment of an absorbent article including an absorbent structure according to the present invention, for instance such as provided by Example 1. It should be understood, however, that the present invention is also applicable to other absorbent articles such as feminine hygiene garments, baby pants, adult incontinent garments and the like.

The absorbent article is shown in its flat out, un-contracted state with the wearer side facing the viewer. Portions of the absorbent article are cut away to more clearly show the underlying structure of the diaper 10 including the absorbent elements and absorbent components. The chassis 12 of the diaper 10 in Figure 6 comprises the main body of the diaper 10. The chassis 12 comprises an outer covering including a liquid pervious top sheet 18 and/or a liquid impervious back sheet 20. The chassis 12 may include a portion of an absorbent structure 14 encased between the top sheet 18 and the back sheet 20. The chassis 12 may also include most or all of the absorbent structure 14 encased between the top sheet 18 and the back sheet 20. The chassis 12 preferably further includes side panels or ears 22, elasticized leg cuffs 24 and elastic waist features 26, the leg cuffs 24 and the elastic waist feature 26 each typically comprise elastic members 28. One end portion of the diaper 10 is configured as a front waist region 30 of the diaper 10. The opposite end portion is configured as a back waist region 32 of the diaper 10. An intermediate portion of the diaper 10 is configured as a crotch region 34, which extends longitudinally between the first and second waist regions 30 and 32. The waist regions 30 and 32 may include elastic elements such that they gather about the waist of the wearer to provide improved fit and containment (e.g. elastic waist feature 26). The crotch region 34 is that portion of the diaper 10 which, when the diaper 10 is worn, is generally positioned between the wearer's legs. The diaper 10 is depicted with its longitudinal axis 36 and its transverse axis 38. The periphery of the diaper 10 is defined by the outer edges of the diaper 10 in which the longitudinal edges 42 run generally parallel to the longitudinal axis 36 of the diaper 10 and the end edges 44 run between the longitudinal edges 42 generally parallel to the transverse axis 38 of the diaper. The chassis 12 also comprises a fastening system, which may include at least one fastening or securing member 46 and at least one landing zone 48. The various components within the diaper 10 may be bound, joined or secured by any method know in the art, for example by adhesives in uniform continuous layers, patterned layers or arrays of separate lines, spirals or spots. The top sheet 18, the back sheet 20, the absorbent structure 14 and other components may be assembled in a variety of well-known configurations and are well known in the art.

The back sheet 20 covers the absorbent structure 14 and preferably extends beyond the absorbent structure 14 toward the longitudinal edges 42 and end edges 44 of the diaper 10 and may be joined with the top sheet 18. The back sheet 20 prevents the bodily exudates absorbed by the absorbent structure 14 and contained within the diaper 10 from soiling other external articles that may contact the wearer, such as bed sheets and undergarments. In preferred embodiments, the back sheet 20 is substantially impervious to bodily exudates and comprises a laminate of a nonwoven and a thin plastic film such as a thermoplastic film. The back sheet 20 may comprise breathable materials that permit vapour to escape from the diaper 10 while still preventing bodily exudates from passing through the back sheet 20. It may be semi-rigid, non-elastic and can be made fully or partially elasticized and include backing. The back sheets 20 may be assembled in a variety of well-known configurations and are well known in the art.

The diaper 10 comprises a top sheet 18 that is preferably soft, compliant, exhibits good strikethroughs and has a reduced tendency to rewet from the liquid absorbent material. The top sheet 18 is placed in close proximity to the skin of the wearer when the diaper 10 is worn. In this way, such top sheet 18 permits bodily exudates to rapidly penetrate it so as to flow toward the absorbent structure 14 more quickly, but preferably not allowing such bodily exudates to flow back through the top sheet 18. The top sheet 18 may be constructed from any one of a wide range of liquid and vapour permeable, preferably hydrophilic, materials. The upper and lower surface of the top sheet 18 may be treated differently and may for instance include a surfactant on the upper surface so as to facilitate liquid transfer there through, especially at a central zone or area of the top sheet 18 located over the absorbent structure 10, and for instance include a hydrophobic agent on the lower surface to minimize the liquid contained within the absorbent core from contact wetting the top sheet 18 thereby reducing rewet values. The top sheet 18 may also be coated with a substance having rash preventing or rash reducing properties (e.g. aloe vera). The top sheet 18 covers substantially the entire wearer facing area of the diaper 10, including substantially all of the front waist region 30, back waist region 32, and crotch region 34. Further, the side panels 22 and/or waist feature layers of the inner region may be formed from the same single top sheet material and, thus, may be referred to as being unitary with the top sheet 18 in forming longitudinal and lateral extensions of the top sheet 18 material. Alternatively, the top sheet 18 may be formed from multiple different materials which vary across the width of the top sheet 18. Such a multiple piece design allows for creation of preferred properties and different zones of the top sheet 18. The top sheet 18 be semi-rigid, non-elastic and can be made fully or partially elasticized. The top sheet 18 may be assembled in a variety of well-known configurations and are well known in the art.

The absorbent structure 14 in Figure 6 generally is disposed between the top sheet 18 and the back sheet 20. The absorbent structure 14 may comprise any absorbent material 110 that is generally compressible, conformable, non-irritating to the wearer's skin, and capable of absorbing and retaining bodily exudates. The absorbent structure 14 may comprise a wide variety of liquid absorbent materials 110 commonly used in absorbent articles such as fluff pulp, which is generally referred to as airlaid. Examples of other suitable absorbent materials include creped cellulose wadding; melt blown polymers; chemically stiffened, modified or cross-linked cellulosic fibres; tissue, including tissue wraps and tissue laminates; absorbent foams; absorbent sponges; absorbent polymer materials; absorbent gelling materials; or any other known absorbent materials or combinations of materials. The absorbent structure 14 may further comprise minor amounts (typically less than 10 %) of non-liquid absorbent materials, such as adhesives, binders, plastics, waxes, oils and the like. The absorbent structure 14 according to various embodiments of the invention may be configured to extend substantially the full length and/or width of the diaper 10. However, alternatively the absorbent structure 14 according to the invention is not coextensive with the entire diaper 10 and is limited to certain regions of the diaper 10 such as for instance the crotch region 34. In various embodiments, the absorbent structure 14 extends to the edges of the diaper 10 and the absorbent material 110 is concentrated in the crotch region 34 or another target zone of the diaper 10. In still another embodiment, the particles can be a combination of absorbent material 110, preferably comprising absorbent polymer material, and skin care particles such as ion exchange resins, deodorant, anti-microbial agents, binder particles, or other beneficial particles.

The diaper 10 may also utilize a pair of containment walls or cuffs 24. Each cuff 24 is a longitudinally extending wall structure preferably positioned on each side of the absorbent structure 14 and spaced laterally from the longitudinal axis 36. The longitudinal ends of the cuffs 24 may be attached or joined, for example, to the top sheet 18 in the front and rear waist regions 30 and 32. Preferably, the ends of the cuffs 24 are tacked down inwardly and attached, for example, by adhesive or sonic bonding to the lower structure. Such a construction effectively biases the cuffs 24 inwardly and is generally considered to cause the cuffs 24 to exhibit improved leakage prevention properties. Preferably, the cuffs 24 are equipped with elastic members 28, which extend along a substantial length of the cuffs 24. In a common application, the elastic members 28 are placed within the cuffs 24, preferably at the top of the cuff 24 while in a stretched condition and then glued or sonic bonded to the cuff 24 at least at their ends. When released or otherwise allowed relaxing, the elastic members 28 retract inwardly. When the diaper 10 is worn, the elastic members 28 function to contract the cuffs 24 about the buttocks and the thighs of the wearer in a manner, which forms a seals between the diaper 10, the buttocks and the thighs. The cuffs 24 may be assembled in a variety of well-known configurations and are well known in the art.

The diaper 10 may also employ additional layers known in the art including an acquisition layer or surge layer, preferably situated between the top sheet and the absorbent core and highloft and/or coverstock layers. This serves to slow down the flow so that the liquid has adequate time to be absorbed by the absorbent core.

In order to keep the diaper 10 in place about the wearer, preferably at least a portion of the back waist region 32 is attached by fastening or securing members 46 to at least a portion of the front waist region 30, preferably to form leg openings and an absorbent article waist. Fastening or securing members 46 carry the tensile load around the absorbent article waist and compliment the elastic members 28 by providing a quasi-seal between the wearer, the elastic waist feature 26 and cuffs 24, so that bodily exudates are contained within the diaper 10 which are then absorbed. In other words, so that it does not leak through gaps between the wearer and the edge of the diaper 10. The fastening or securing members 46 may for instance be adhesive, mechanical fasteners, hook and loop features, conceivable strings and/or combinations thereof, i.e., anything that will secure one end of the diaper 10 to the longitudinally opposite end of the diaper 10. The fastening or securing members 46 may also be co-adhesive such that they adhere to each other but not other materials. The fastening or securing members 46 and any component thereof may include any material suitable for such a use, including but not limited to plastics, films, foams, non-woven webs, woven webs, paper, laminates, fibre reinforced plastics and the like, or combinations thereof. It may be preferable that the materials making up the fastening or securing members 46 are flexible, extensible and/or elastic, allowing them to better conform to the shape and movements of the body and thus, reduces the likelihood that the fastening system will irritate or injure the wearer's skin. Preferably, the diaper 10 is affixed to the wearer by tape fasteners which are permanently affixed to the back sheet 20. Tape fasteners are contacted with the transversely opposite side panel or ears 22 attached or joined and extending from the back sheet 20, where they remain affixed due to the binding compound applied to the fasteners. Alternatively, the absorbent article may be pants and the like. In this configuration, the absorbent article may or may not have tape fasteners. Specific disposability tapes may however also be provided on such absorbent articles. All fastening and securing elements 46 may be assembled in a variety of well-known configurations and are well known in the art.

The waist regions 30 and 32 each comprise a central region and a pair of side panels or ears 22 which typically comprise the outer lateral portions of the waist regions. These side panels 22 may be unitary with the chassis 12 and/or back sheet 20 or may be attached or joined thereto by any means know in the art. In a preferred embodiment of the present invention, the side panels 22 positioned in the back waist region 32 are flexible, extensible and/or elastic in at least the lateral direction (i.e., elasticized side panels), in another embodiment the side panels 22 are non-elastic, semi-rigid, rigid and/or stiff. This variety of side panels 22 are well known in the art.

Furthermore waistbands 26 employing elastic members can be positioned along the transverse portion of the diaper 10 so that when worn, the waistbands 26 are positioned along the waist of the wearer. Generally, the waistband 26 preferably creates a seal against the waist so that bodily exudates do not leak from the regions between the elastic waistband 26 and the waist of the wearer. Although the bodily exudates are primarily absorbed by the absorbent materials within the diaper 10, the seal is important considering the assault of liquid by the wearer may overwhelm the absorption rate capacity of the absorbent structure 14. Hence, the waistbands 26 contain the liquid while it is being absorbed, they are well known in the art.

The absorbent article such as a diaper 10 may also include such other features, components and elements as are known in the art including front and rear ear panels, waist cap features, elastics and the like to provide better fit, containment and aesthetic characteristics. These features may be assembled in a variety of well-known configurations and are well known in the art.

## Claims

1. An absorbent structure for use in an absorbent article comprising:
a) a distribution layer having an absorbent capacity of at least about 5 g/m2;
b) an immobilisation layer which is joined to the distribution layer to define compartments there between; and
c) an absorbent material held in at least one of the compartments:
i) wherein said absorbent material comprises an absorbent polymer material, and
ii) from zero to an amount less than about 40 weight percent absorbent fibrous material, based on the weight of absorbent polymer material.

2. Absorbent structure according to claim 1, wherein the distribution layer is provided by a substantially continuous layer comprising absorbent cellulosic fibres, paper, tissue, airlaid, drylaid or wetlaid material.

3. Absorbent structure according to claim 1 or 2, wherein the immobilisation layer is a thermoplastic adhesive, non-woven, polymer film, tissue, paper, airlaid, drylaid or wetlaid layer.

4. Absorbent structure according to any of claims 1 to 3, wherein the absorbent material comprises an absorbent polymer material.

5. Absorbent structure according to any of claims 1 to 4, wherein the absorbent polymer material is present in the absorbent material in an average basis weight of at least 100 g/m².

6. Absorbent structure according to any of claims 1 to 5, wherein the joints between the lower surface of the immobilizing layer and the upper surface of the distribution layer are not permanent so that the joints partially loosen during wetting.

7. Absorbent structure according to any of claim 1 to 6, wherein the absorbent structure has a flexure-resistance of less than 500.0 grams, more preferably less than 250.0 grams, and still more preferably less than about 175.0 grams and most preferably less than 130.0 grams or 100.0 grams.

8. Absorbent structure according to any of claims 1 to 7, with a wet immobilization of more than 50 wt%

9. An absorbent article comprising a liquid pervious topsheet, a liquid impervious backsheet and a liquid absorbing absorbent structure situated in between the liquid pervious topsheet and liquid impervious backsheet according to any of the above claims 1 to 8.

10. Absorbent article according to claim 9, having a crotch width of less than 90 mm.

11. Absorbent article according to claim 9 or 10, wherein the absorbent structure is provided with a cover layer comprising a non-woven material, cellulosic fibres, paper, tissue, airlaid, drylaid or wetlaid material.

12. Absorbent article according to any of claims 9 to 11, wherein portions of the cover layer join to portions of the distribution layer via the immobilisation layer; the distribution layer together with the cover layer thereby providing cavities for the immobilization of the absorbent polymer material.

13. Absorbent article according to claim 12, wherein the portions of the cover layer joined to the portions of the distribution layer provide areas of junction, said areas of junction are arranged longitudinally or transversally.

14. Method for the manufacturing of an absorbent structure for use in an absorbent article comprising the steps of:
- providing a distribution layer having an absorbing capacity of at least about 5 g/m2;
- covering the distribution layer with an absorbent material wherein said absorbent material comprises
i) an absorbent polymer material, and
ii) from zero to an amount less than about 40 weight percent absorbent fibrous material, based on the weight of absorbent polymer material;
- covering the absorbent material with an immobilisation layer which is joinable to the distribution layer; and
- joining the immobilisation layer to the distribution layer to define compartments there between wherein the absorbent material is held in at least one of the compartments.
